Europäisches Patentamt

(19) European Patent Office

Office européen des brevets

(11) EP 0 765 122 B1

## (12) FASCICULE DE BREVET EUROPEEN

(45) Date de publication et mention
de la délivrance du brevet:
**14.04.1999 Bulletin 1999/15**

(21) Numéro de dépôt: **95923386.7**

(22) Date de dépôt: **14.06.1995**

(51) Int. Cl.$^6$: **A21D 8/04**, C12N 1/18,
C12N 1/04

(86) Numéro de dépôt international:
**PCT/FR95/00783**

(87) Numéro de publication internationale:
**WO 95/34214 (21.12.1995 Gazette 1995/54)**

(54) **PROCEDE DE FABRICATION D'UN LEVAIN DE PANIFICATION DESHYDRATE ET LEVAIN OBTENU**

VERFAHREN ZUR HERSTELLUNG VON DEHYDRIERTEM SAUERTEIG UND SO ERHALTENER SAUERTEIG

METHOD FOR PRODUCING A DEHYDRATED SOURDOUGH, AND SOURDOUGH THUS OBTAINED

(84) Etats contractants désignés:
**AT BE CH DE DK ES FR GB GR IE IT LI NL PT**

(30) Priorité: **14.06.1994 FR 9407245**

(43) Date de publication de la demande:
**02.04.1997 Bulletin 1997/14**

(73) Titulaire: **EUROGERM S.A.**
**21800 Quetigny (FR)**

(72) Inventeurs:
• **GERVAIS, Patrick**
**21800 Quetigny (FR)**
• **HERAIL-FOUSSEREAU, Claude**
**21800 Quetigny (FR)**
• **MARECHAL, Pierre André**
**21000 Dijon (FR)**

• **FASQUEL, Jean-Philippe**
**21560 Arc-sur-Tille (FR)**
• **GIRARD, Jean-Philippe**
**21121 Fontaine-les-Dijon (FR)**

(74) Mandataire: **Bruder, Michel**
**Cabinet Claude Guiu**
**10, rue Paul Thénard**
**21000 Dijon (FR)**

(56) Documents cités:
**EP-A- 0 339 750        FR-A- 2 148 727**
**FR-A- 2 197 977**

• **DATABASE WPI Section Ch, Week 7822 Derwent Publications Ltd., London, GB; Class D16, AN 78-39840A & SU,A,564 495 (KAZA POLY) , 22 Août 1977**

**Description**

La présente invention concerne un procédé de fabrication d'un levain de panification déshydraté, dans lequel la flore microbienne présente une viabilité élevée de la levure, et levain ainsi obtenu.

Dans toute la suite, l'expression "levain" désigne une pâte essentiellement à base de farine, d'eau potable, de sel et d'une flore ou biomasse microbienne, biomasse de panification, capable de déclencher la fermentation du pain.

Ce terme s'applique également aux produits obtenus par déshydratation du levain tel que défini précédemment, sous réserve qu'ils contiennent une flore microbienne vivante en nombre suffisant pour obtenir la levée du pain. Pour produire un levain sec, il est donc nécessaire que la biomasse microbienne déshydratée survive dans un état de dormance pendant le stockage et soit ensuite réactivée par la réhydratation. Cette biomasse est constituée de bactéries acidifiantes lactiques et acétiques ainsi que de levures spécifiques du levain.

Pour maintenir une viabilité relativement élevée des cellules dans le levain sec, il est connu d'ajouter un adjuvant de séchage tel que polysaccharides, émulsifiants. Toutefois, ces adjuvants de séchage présentent un inconvénient majeur car ils sont nuisibles à la qualité de la pâte obtenue.

On connaît par ailleurs, par le document FR-A-2.197.977 un procédé de déshydratation de levain de panification sans adjuvant de séchage consistant en un broyage de levure fraîche sous une forme pulvérulente qui peut être facilement séchée ; toutefois les températures de séchage sont comprises entre 32 et 55°C.

On connaît par le document EP-A-0.339.750 un autre procédé de déshydratation en deux étapes successives :

\*   1ère étape : addition successive de différents mélanges (farine + eau) jusqu'à atteindre un produit contenant de l'ordre de 20 à 35% d'humidité.

 Ces additions permettent de modifier progressivement la composition d'un levain de panification (farine, eau, levures et bactéries), initialement liquide, jusqu'à l'amener sous une forme solide, particulaire, permettant de la déshydrater par air chaud.
 La particularité du procédé consiste à additionner des mélanges (farine + eau) contenant de moins en moins d'eau, afin de diminuer la teneur en eau globale du système, mais autorisant une poursuite des activités fermentaires afin de conserver dans le levain en poudre des concentrations microbiennes proches de celles du levain liquide initial.

\*   2ème étape : séchage par courant d'air sec et chaud (température inférieure à 65°C) pour atteindre entre 5 et 12% d'humidité finale, en 2 à 16 heures selon le débit d'air et la température de l'air. Le séchage n'est pas contrôlé.

Le levain décrit, est constitué par des cellules viables d'une levure et d'une bactérie, de farine de grains entiers et de 5 à 12% d'eau. Des adjuvants de séchages peuvent être éventuellement ajoutés.La présente invention vise à remédier à ces inconvénients en procurant un procédé de déshydratation d'un levain de panification qui conserve après la déshydratation, une viabilité relativement élevée des cellules de la biomasse utilisée, et ce, sans faire appel à un quelconque adjuvant; l'addition de farine et d'eau a lieu en une fois et conduit à un levain liquide qui est alors soumis à la déshydradation. Le séchage à l'air est réalisé en deux étapes dont la vitesse est réglée par le contrôle de l'humidité de l'air. Bien entendu, il n'est pas obligatoire d'associer une levure et une bactérie, comme dans le document antérieur.

A cet effet, ce procédé de fabrication d'un levain de panification déshydraté dans lequel on mélange de façon homogène une biomasse de panification avec un milieu de déshydratation à base de farine et d'eau, pour donner un levain humide, est caractérisé en ce qu'on déshydrate le levain humide à température réduite, c'est-à-dire inférieure à 40°C et en tous cas suffisamment basse pour ne pas détruire la biomasse entrant dans le mélange, en deux étapes successives. La première étape qui correspond à l'enlèvement de l'eau extracellulaire se fait à une vitesse de déshydratation quelconque, tant que l'activité de l'eau $a_w$ est supérieure ou égale à 0,990, c'est-à-dire tant que l'$a_w$ du milieu externe est supérieure à l'$a_w$ intracellulaire des levures, et ensuite, dès que l'activité de l'eau $a_w$ tombe à une valeur inférieure à 0,990 (correspondant à un potentiel hydrique de -1,38 MPa) on déshydrate le levain avec un gradient de potentiel hydrique contrôlé, correspondant à des vitesses de déshydratation comprises entre -0,5kPa/s et -5,0kPa/s jusqu'à ce que l'activité de l'eau $a_w$ atteigne une valeur correspondant à une viabilité cellulaire satisfaisante, cette valeur pouvant tomber jusqu'à 0,15 (correspondant à un potentiel hydrique de -261 MPa). Il est à noter que l'intensité de la vitesse de déshydratation est donnée par la valeur absolue de ce paramètre (une vitesse de -5,0kPa/s est donc considérée comme plus élevée qu'une vitesse de -0,5kPa/s).

On rappelle que l'activité de l'eau $a_w$ est donnée par la formule de Norrish extraite du "Journal of Food Technology" :

2

$$A_W = 1 - (Xs)\ e^{\ (-KX_S)^2}$$

où K est une constante dépendant du soluté (1,16 pour le glycérol) et Xs, la fraction molaire du soluté.

Cette technique permet de produire un levain déshydraté complet, au sens boulanger du terme, c'est-à-dire

- contenant suffisamment de levures pouvant être revivifiées, pour provoquer une levée correcte de la pâte ;
- contenant une flore de levures lactiques pouvant être revivifiées, capable d'assurer la production des acides organiques (lactique et acétique principalement) responsables de la saveur du pain au levain. Afin d'obtenir des taux de survie de la flore microbienne élevés, plus spécialement d'au moins 50 % , et même de 60 %, voire de 70 %, on applique une cinétique lente de variation de la pression osmotique au cours de la deuxième déshydratation jusqu'à l'obtention de la pression osmotique finale qui doit être obtenue en fonction de l'$a_w$ recherchée.

L'utilisation de rampes osmotiques permet avantageusement le contrôle d'une telle cinétique comme illustré par les exemples.

De manière avantageuse, au moins la deuxième étape de déshydratation est réalisée à l'intérieur d'une étuve dont on fait varier l'humidité relative par paliers réguliers depuis une valeur d'environ 95 % jusqu'à une valeur finale de 15% au plus lorsque $a_w$ tombe à 0,15. On notera que dans les dernières phases de séchage, on utilise avec avantage un dessiccateur produisant un air dont l'humidité relative est proche de 0%.

La température réduite de déshydratation est choisie inférieure à 40°C et, de préférence, comprise entre 20 et 25°C.

L'évaluation de l'activité de l'eau $a_w$ du levain est déduite de l'évaluation de la température de rosée mesurée par l'apparition de buée sur un miroir, ou, en variante, par pesées régulières du mélange levure/farine déterminant la teneur en eau et donc l'activité de l'eau $a_w$ déduite de l'isotherme de sorption, établie pour le produit à la température de l'opération.

On réalise de préférence la culture de biomasse dans un milieu hyperosmotique afin de favoriser la synthèse intracellulaire de solutés protecteurs ou encore dans un milieu enrichi en sucre pour favoriser l'accumulation de solutés protecteurs.

La biomasse mise en oeuvre dans le procédé de l'invention est essentiellement à base de levures et/ou de bactéries capables d'assurer la fermentation du pain dans des conditions satisfaisantes.

De manière classique, les levures appartiennent au genre Saccharomyces, l'espèce S.cerevisiae étant le plus largement utilisée, et les bactéries du genre Lactobacillus, comme par exemple L. plantarum, étant entendu que d'autres souches de levures et de bactéries sont utilisables.

L'invention vise également les levains déshydratés tels qu'obtenus par le procédé défini ci-dessus. Ces levains déshydratés sont donc caractérisés en ce qu'ils sont dépourvus d'adjuvants de séchage et que le taux de viabilité cellulaire est d'au moins 50 % , et même de 60 %, voire de l'ordre de 70 %, pouvant atteindre et dépasser 100%, compte tenu du developpement éventuel de la flore bactérienne pendant la première étape de déshydratation.

D'autres caractéristiques et avantages de l'invention sont donnés dans les exemples qui suivent concernant la déshydratation d'un levain humide et de bactéries et en se reportant aux figures 1 à 3, qui représentent respectivement :

- la figure 1, un diagramme donnant, en fonction du temps, la variation de l'activité de l'eau $a_w$ d'un levain de panification ( représentée par des croix sur le dessin) et l'humidité relative HRE (représentée en traits continus sur le dessin) de l'ambiance dans l'étuve industrielle,
- la figure 2, une représentation de la vitesse de déshydratation du levain en fonction du temps, et,
- la figure 3, l'isotherme de sorption du levain.

I - Procédé de déshydratation d'un levain de panification.

On utilise un levain humide élaboré à partir de 70 g d'eau, 30 g de farine et 0,5 g d'une levure Saccharomyces cerevisiae (souche CBS 1171).

On a avantageusement procédé, avant la déshydratation, à une culture comme indiqué plus haut de la levure dans un milieu hyperosmotique. Les expérimentations ont été effectuées dans une étuve industrielle Hereauss à humidité relative contrôlée dans la gamme 40% - 95 %, à une température constante de 25°C.

Au cours de la première étape de déshydratation du levain, on a maintenu, à l'intérieur de l'étuve, une humidité relative de 40 %, par exemple pendant une heure, jusqu'à ce que l'activité de l'eau $a_w$ tombe à la valeur 0,990. Au cours de cette première étape de déshydratation le gradient de potentiel hydrique est très élevé et provoque un départ d'eau très important et la vitesse de déshydratation, exprimée en kPa/s augmente progressivement.

Aussitôt que l'activité de l'eau du levain tombe à la valeur de 0,990, on augmente brutalement et fortement l'humidité relative dans l'ambiance, pour la porter à 95%, puis on la fait décroître par paliers réguliers, par exemple de 5% toutes les deux heures, jusqu'à la valeur de 60% au bout d'une période de temps variable déterminant la vitesse de déshydratation. On arrête l'opération de déshydratation lorsque la valeur de l'activité de l'eau $a_w$ tombe à 0,60. On obtient ainsi, au cours de la deuxième étape du procédé de déshydratation, une vitesse de déshydratation relativement lente qui est comprise entre -0,5kPa/s et -5kPa/s.

Au cours des essais, l'évaluation de l'activité de l'eau $a_w$ du levain pendant la phase de déshydratation a été effectuée par deux méthodes différentes menées parallèlement :

- Par mesure directe de l'activité de l'eau $a_w$,

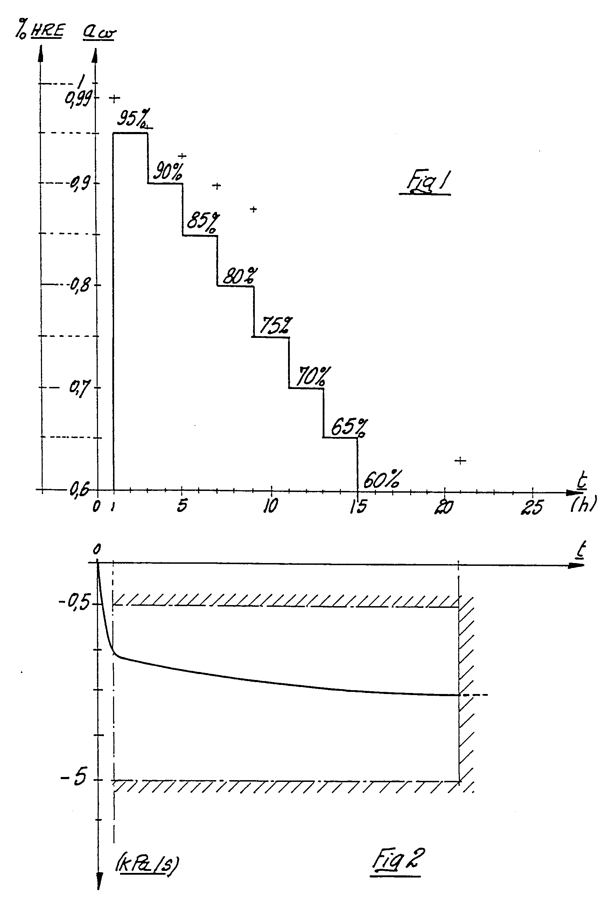

  pour chaque relevé permettant d'établir la figure 1, il a été prélevé dans l'étuve un échantillon représentatif du produit dont on peut facilement déduire une évaluation de la température de rosée mesurée par l'apparition de buée sur un miroir ; on a eu recours pour cette évaluation à un appareil du type CS-2, DECAGON-USA. A noter que le temps nécessaire pour effectuer une telle mesure est de l'ordre de 3 à 5 minutes par échantillon.

- Par mesure indirecte à partir de l'isotherme de sorption du mélange que l'on étudie. On rappelle que l'isotherme de sorption est une courbe reliant les deux paramètres teneur en eau/activité de l'eau ; cet isotherme est bien entendu spécifique à chaque produit.

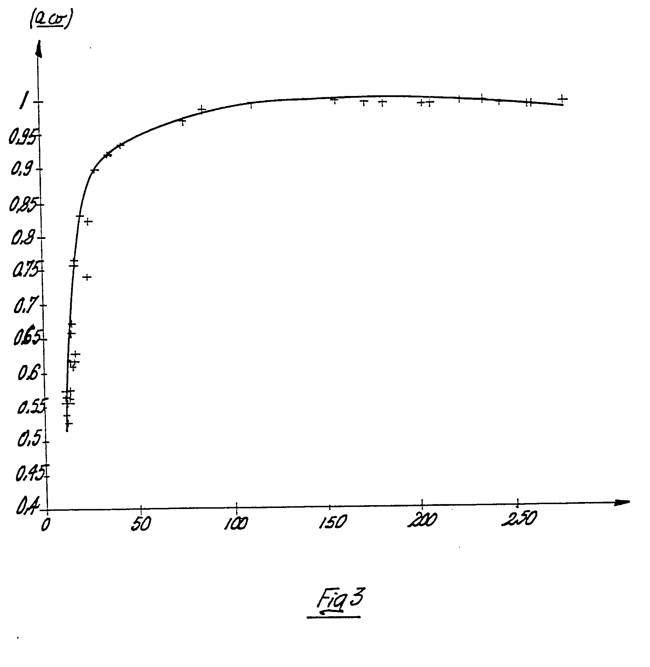

On a représenté en figure 3 l'isotherme de sorption du mélange ("activité de l'eau" $a_w$ en fonction de la "teneur en eau" exprimée en grammes d'eau pour 100 grammes de matière sèche) dont on a décrit ci-dessus les phases de déshydratation. A partir de l'isotherme de sorption du mélange considéré (0,5 gr de levure, 30 gr de farine) et connaissant la teneur en eau initiale dudit mélange, il est évidement possible de suivre très simplement les variations de l'activité de l'eau par mesure régulière de la masse du mélange. On observera qu'une telle pesée est relativement aisée, dans la mesure où l'on travaille à 25°C, c'est-à-dire sensiblement à la température ambiante.

Enfin, il est à noter que le suivi de l'activité de l'eau par l'une ou l'autre des méthodes qui viennent d'être décrites, conduit naturellement à la connaissance du potentiel hydrique qui lui correspond selon la formule connue :

$$\text{potentiel hydrique (Pa)} = RT/V_w.\ln a_w$$

dans laquelle R est la constante des gaz parfaits ($8,314 \text{ J.mol}^{-1}.\text{K}^{-1}$) et T la température (°K) et $V_w$ est le volume molaire partiel de l'eau (à savoir $1,8.10^{-5} \text{ m}^3.\text{mol}^{-1}$).

Comme il a été indiqué précédemment, aucun adjuvant de déshydratation n'est incorporé pendant les deux phases de déshydratation à l'intérieur de l'étuve. On procède ensuite à une mesure du pourcentage de viabilité et les résultats obtenus par le procédé suivant l'invention, mis en oeuvre avec une vitesse de déshydratation lente correspondant à -1kPa/s, une vitesse de déshydratation intermédiaire correspondant à -3,75kPa/s et une vitesse de déshydratation élevée correspondant à -4,17 kPa/s sont donnés dans le tableau 1 ci-dessous. Les résultats indiqués dans le tableau 1 confirment l'influence de la vitesse de déshydratation de la dernière étape sur la viabilité cellulaire, à savoir : plus la déshydratation est lente, meilleure est la conservation de la viabilité.

Tableau 1

| Type d'essai | Vitesse de déshydratation | % viabilité avant traitement | % viabilité après traitement | % perte viabilité |
|---|---|---|---|---|
| Essai rapide | -4,17 kPa/s | 97,54 | 48,08 | 49,46 |
| Essai interméd. | Etuvage -3,75 kPa/s | 97,54 | 56,96 | 40,58 |
| Essai lent | Etuvage -1 kPa/s | 97,54 | 68,55 | 29,00 |

II - Procédé de déshydratation de bactéries de levain.

- déshydratation des bactéries jusqu'à l'obtention de $a_w = 0,29$

a) Microorganisme et conditions de culture

L.plantarum L-73 est cultivée en milieu MRS (De Man et al, 1960, Journal of Applied Bactériology 23(1), p. 130 - 135)) à 30°C, sous atmosphère d'azote.

La pression osmotique de ce milieu est ajustée à -1,38 MPa à l'aide de glycérol ($a_w$ = 0,990).

b) Choc osmotique

Après une pré-culture de 12 h, suivie d'une culture de 24 h, en milieu MRS, 9 ml de suspension bactérienne sont centrifugés (5 min ; 2860 g).

Le culot cellulaire est alors remis en suspension à l'aide d'une solution eau-glycérol de pression osmotique connue ($\pi$ = 171 MPa ; $a_w$ = 0,290).

c) Rampes osmotiques

Après une pré-culture de 12 h, suivie d'une culture de 24 h, en milieu MRS ($a_w$ = 0,990), 9 ml de suspension bactérienne sont centrifugés. Le culot cellulaire est alors remis en suspension dans 9 ml d'une solution eau-glycérol, à une pression osmotique de -1,38 MPa. 1 ml de cette suspension est mélangé à 3 ml d'un mélange eau-glycérol ($a_w$ = 0,990). 43 ml de glycérol pur sont ajoutés à différentes cinétiques par l'intermédiaire d'un pousse-seringue (Melsungen, Allemagne ; Razel, Etat-unis), afin d'atteindre une pression osmotique finale de 171 MPa ($a_w$ = 0,29).

Quatre cinétiques de variation de la pression osmotique sont testées :

0,0209 MPa/s (135 min)
0,0565 MPa/s (50 min)
0,1178 MPa/s (24 min)
0,2570 MPa/s (11 min)

d) Mesure de la viabilité cellulaire

La viabilité cellulaire est déterminée selon la méthode U.F.C. (unité formant colonie). Les dilutions sont réalisées dans une solution eau-glycérol de pression osmotique identique à la pression osmotique finale (171 MPa).

Pour chaque dilution, 100 µl sont inoculés sur milieu MRS agar ($\pi$ = -1,38 MPa ; $a_w$ = 0,990) (3 boîtes par dilution).

Les boîtes sont incubées 48 h à 30°C sous atmosphère d'azote.

Le taux de viabilité de chaque traitement est comparé à un contrôle, maintenu à la même pression osmotique ($\pi$ = -1,38 MPa), mais également dilué.

Les résultats obtenus sont donnés dans le tableau suivant :

| | | Rampes (Mpa/s) | | | |
|---|---|---|---|---|---|
| | Choc | 0,2570 (11 min) | 0,1178 (24 min) | 0,0565 (50 min) | 0,0209 (135 min) |
| % de viabilité | 43% | 73% | 86% | 88% | 83% |
| | 40% | 78% | 87% | 90% | 85% |
| | 40% | 78% | 91% | 93% | 88% |

L'examen de ces résultats montre que l'augmentation de la pression osmotique du milieu externe par rampes permet d'augmenter significativement le taux de survie des bactéries obtenu lors du choc.

- déshydratation des bactéries jusqu'à l'obtention de $a_w$ = 0,17.

On opère comme indiqué ci-dessus, mais on effectue le choc osmotique en appliquant une pression de -245 MPa, l'$a_w$ étant de 0,170. En outre, dans l'étape c), on mélange 1 ml de la suspension cellulaire avec 54 ml de glycérol pur, afin d'atteindre une pression osmotique finale de -245 Mpa ($a_w$ = 0,170).

Les résultats donnés dans le tableau suivant correspondent aux 2 cinétiques de variation de la pression osmotique indiquées ci-après :

- 0,0255 MPa/s (160 min)
- 0,0864 MPa/s (47 min).

Lors de la mesure de la viabilité cellulaire, selon l'étape d), on réalise des dilutions dans une solution eau-glyrérol de pression osmotique identique à la pression finale (- 245 MPa).

| Rampes (Mpa/s) | | | |
|---|---|---|---|
| | Choc | 0,0864 MPa/s (47 min) | 0.0255 MPa/s (260 min) |
| % de viabilité | 17% | 39% | 52%% |
| | 13% | 40% | 56% |
| | 16% | Non effectué | 46% |

Ainsi, comme dans l'exemple précédent, on constate que l'application d'une cinétique lente de variation de la pression osmotique permet d'augmenter le taux de viabilité des cellules, par rapport à celui obtenu avec des cinétiques plus rapides ou en soumettant les cellules à un choc osmotique.

**Revendications**

1. Procédé de fabrication d'un levain de panification déshydraté dans lequel on mélange de façon homogène une souche de levure avec un milieu de déshydratation élaboré à partir de farine et d'eau, pour donner un levain humide, *caractérisé* en ce qu'on déshydrate le levain humide à température , inférieure à 40°C pour éviter la destruction de la biomasse entrant dans le mélange, en deux étapes successives, d'abord à une vitesse de déshydratation quelconque, tant que l'activité de l'eau $a_w$ est supérieure ou égale à 0,990 et ensuite, dès que l'activité de l'eau $a_w$ tombe à une valeur inférieure à 0,990 on déshydrate le levain avec un gradient de potentiel hydrique contrôlé correspondant à des vitesses de déshydratation comprises entre -0,5kPa/s et -5,0kPa/s jusqu'à ce que l'activité de l'eau $a_w$ atteigne une valeur correspondant à une viabilité cellulaire satisfaisante, cette valeur pouvant tomber jusqu'à 0,15.

2. Procédé selon la revendication précédente, *caractérisé* en ce qu'on applique une cinétique lente de la variation de la pression osmotique au cours de la deuxième étape de déshydratation, de manière à obtenir des taux de survie de la biomasse microbienne au moins de 50%, et même de 60%, voire de 70%.

3. Procédé selon l'une quelconque des revendications précédentes, *caractérisé* en ce que la deuxième étape de déshydratation au moins, est réalisée à l'intérieur d'une étuve dont on fait varier l'humidité relative par paliers réguliers depuis une valeur de 95% jusqu'à une valeur finale de 15% au plus lorsque $a_w$ tombe à 0,15.

4. Procédé selon l'une quelconque des revendications précédentes, *caractérisé* en ce qu'on opère à une température de 20 à 25°C, notamment de 25°C.

5. Procédé selon l'une des revendications 1 à 4, *caractérisée* en ce que l'évaluation de l'activité de l'eau $a_w$ du levain est déduite de l'évaluation de la température de rosée mesurée par l'apparition de buée sur un miroir.

6. Procédé selon l'une quelconque des revendications 1 à 4 *caractérisé* en ce que l'évaluation de l'activité de l'eau $a_w$ du levain est effectuée par pesées régulières du mélange levure/farine déterminant la teneur en eau et donc l'activité de l'eau $a_w$ déduite de l'isotherme de sorption, établie pour le produit à la température de l'opération.

7. Procédé selon l'une quelconque des revendications précédentes *caractérisé* en ce que la biomasse est à base de levure et/ou de bactéries capables d'assurer la fermentation du pain, ces levures et bactéries étant avantageusement respectivement choisies dans le genre Saccharomyces, telle que S.cerevisiae et dans le genre Lactobacillus, telle que L.plantarum.

## Claims

1. Process for the production of a dehydrated leaven for bread-making, in which a yeast strain is mixed uniformly with a dehydrating medium composed of flour and water to give a wet leaven,

    **characterized in that**

    the wet leaven is dehydrated at a temperature lower than 40°C to avoid destruction of the biomass in the mixture; in two successive stages, first at an arbitrary dehydration rate such that the water activity $a_w$ remains greater than or equal to 0.990 and then, once the water activity $a_w$ has fallen to a value below 0.990, the leaven is dehydrated at a controlled hydrous potential gradient corresponding to dehydration rates between -0.5kPa/s and -5.0kPa/s, until the water activity $a_w$ reaches a value at which cell viability remains satisfactory but which may be as low as 0.15.

2. Process according to the preceding claim,

    **characterized in that**

    the kinetics of the osmotic pressure variation are made slow during the second dehydration phase, so as to obtain microbiotic biomass survival rates of at least 50% and even as high as 60% or 70%.

3. Process according to either of the preceding claims,

    **characterized in that**

    at least the second dehydration stage is carried out in a stove whose internal relative humidity is varied in regular steps from a value of 95% down to a final value of at most 15%, when $a_w$ falls to 0.15.

4. Process according to any of the preceding claims,

    **characterized in that**

    the working temperature is 20° to 25°C, notably 25°C.

5. Process according to any of Claims 1 to 4,

    **characterized in that**

    the water activity $a_w$ of the leaven is evaluated by deducing it from a measurement of the dew-point determined by the appearance of mist on a mirror.

6. Process according to any of Claims 1 to 4,

    **characterized in that**

    the water activity $a_w$ of the leaven is evaluated gravimetrically by regular weighing of the yeast/flour mixture to determine its water content and hence the water activity $a_w$ deduced from the sorption isotherm established for the product at the working temperature.

7. Process according to any of the preceding claims,

    **characterized in that**

    the biomass is based on yeast and/or bacteria capable of generating fermentation in the bread, the said yeasts and bacteria being advantageously chosen, respectively, from the Saccharomyces genus, such as *S. cerevisiae* and from the Lactobacillus genus, such as *L. plantarum*.

## Patentansprüche

1. Herstellungsverfahren für eine entwässerte Brotbereitungshefe, bei dem man in homogener Weise einen Hefestamm mit einem aus Mehl und Wasser hergestellten Entwässerungsmilieu vermischt, um eine feuchte Hefe hervorzubringen, dadurch gekennzeichnet, daß man die feuchte Hefe bei einer Temperatur unter 40°C, um die Zerstörung der in die Mischung eingehenden Biomasse zu verhindern, in zwei aufeinanderfolgenden Schritten entwässert, zuerst bei einer beliebigen Entwässerungsgeschwindigkeit, solange die Aktivität des Wassers $a_w$ größer oder gleich 0,990 ist, und dann, sobald die Aktivität des Wassers auf einen Wert kleiner 0,990 sinkt, die Hefe mit einem gesteuerten Wasserpotentialgradienten, entsprechend Entwässerungsgeschwindigkeiten, die zwischen -0,5 kPa/s und -5,0kPa/s liegen, entwässert, bis die Aktivität des Wassers $a_w$ einen Wert erreicht, der einer zufriedenstellenden Zell-Lebensfähigkeit entspricht, wobei dieser Wert bis auf 0,15 sinken kann.

2. Verfahren nach dem vorangehenden Anspruch, dadurch gekennzeichnet, daß man im Verlauf des zweiten Entwässerungsschritts eine langsame Kinetik der Veränderung des osmotischen Drucks anwendet, um Überlebensraten der mikrobiellen Biomasse von mindestens 50 %, selbst von 60 %, ja sogar von 70 % zu erhalten.

3. Verfahren nach einem beliebigen der vorangehenden Ansprüche, dadurch gekennzeichnet, daß mindestens der zweite Entwässerungsschritt im Inneren eines Trockenofens durchgeführt wird, dessen relative Feuchtigkeit man in regelmäßigen Stufen von einem Wert von 95 % bis zu einem Endwert von höchstens 15 %, wenn $a_w$ auf 0,15 sinkt, verändert.

4. Verfahren nach einem beliebigen der vorangehenden Ansprüche, dadurch gekennzeichnet, daß man bei einer Temperatur von 20 bis 25°C, insbesondere von 25°C arbeitet.

5. Verfahren nach einem der Ansprüche 1 bis 4, dadurch gekennzeichnet, daß die Bestimmung der Aktivität des Wassers $a_w$ der Hefe aus der Bestimmung der Temperatur von Tau gemessen durch das Auftreten von Wasserdampf auf einem Spiegel abgeleitet wird.

6. Verfahren nach einem beliebigen der Ansprüche 1 bis 4, dadurch gekennzeichnet, daß die Bestimmung der Aktivität des Wassers $a_w$ der Hefe durch regelmäßige Wägungen der Hefe/Mehl-Mischung durchgeführt wird, wobei der Wassergehalt und damit die aus der Sorptionsisotherme abgeleitete Aktivität des Wassers $a_w$ bestimmt wird, die bei der Arbeitstemperatur für das Produkt etabliert worden ist.

7. Verfahren nach einem beliebigen der vorangehenden Ansprüche, dadurch gekennzeichnet, daß die Biomasse auf Hefe und/oder Bakterien basiert, die imstande sind, die Fermentation von Brot sicherzustellen, wobei diese Hefen und Bakterien vorteilhafterweise in der Gattung Saccharomyces, wie beispielsweise S. cerevisiae, bzw. in der Gattung Lactobazillus, wie beispielsweise L. plantarum, ausgewählt werden.

Fig 1

Fig 2

9

_Fig 3_